# EUROPEAN PATENT APPLICATION

(11) **EP 2 803 628 A1**
(43) Date of publication of application: **19.11.2014**
(21) Application number: 13167933.4
(22) Date of filing: 15.05.2013
(51) Int. Cl.: B67C 3/22, B08B 9/32, F16K 11/076

(54) **Manifold for conveying a sterilizing fluid inside empty articles**

(71) Applicant: Sidel S.p.a. Con Socio Unico, Parma (IT)
(72) Inventor: Quagliarella, Pierluigi, 43100 PARMA (IT)
(74) Representative: Di Sciuva, Michele

(57) **Abstract**

There is disclosed a manifold (10) for conveying a sterilizing fluid inside empty articles (2) movable about an axis (A), comprising: a stator (25) stationary relative to axis (A) and defining at least one duct (26, 28) for sterilizing agent; a rotor (35) defining a second duct (40, 41) and a third duct (41, 40); supporting means (60, 61) radially interposed between a first element (28) of stator (25) and a second element (37) of rotor (35); and a mask (50) fluidly interposed between first duct (26, 28) and one (40) of second duct (40, 41) and third duct (41, 40) on the basis of the angular position of rotor (35) about axis (A), so as to prevent sterilizing agent from flowing inside one (40) of second duct (40, 41) and third duct (41, 40); mask (50) is configured to allow sterilizing agent to flow from first duct (26, 28) to the other one (41) of second duct (40, 41) and third duct (41, 40) on the basis of the angular position of rotor (35) about axis (A); first element (28) is radially internal to second element (37) .

## Description

The present invention relates to a manifold for conveying a sterilizing fluid inside empty articles, in particular containers filled with a pourable food product.

Article-handling machines are known which comprise a plurality of units for carrying out respective operations on articles, in particular containers filled with a pourable food product.

In greater detail, the known article-handling machines substantially comprise:
- a sterilizing unit for sterilizing empty containers;
- a rinsing unit for rinsing containers;
- a filling unit for filling containers with a pourable food product;
- a capping unit for capping the containers; and
- a labelling unit for applying labels onto respective containers.

The sterilizing unit is adapted to inject a sterilizing agent, in particular a solution of peracetic acid, inside the empty containers and upstream of the rinsing unit.

In detail, the sterilizing unit comprises:
- one or more first wheels which rotate each about an axis, and comprise each a plurality of jaws movable between an open position in which they receive/release respective containers and a closed position in which they convey the containers;
- one or more second wheels, known as distributor, which is coaxial with and arranged below the respective first wheel, and which comprises a plurality of nozzles arranged below respective jaws of the respective first wheel;
- a source of the sterilizing agent; and
- a manifold which is fluidly interposed between the source and the nozzles.

The first wheel is fed with containers to be sterilized at an inlet station, conveys the container along an arc-shaped path around the axis, and feeds an outlet station with the sterilized containers.

The containers are conveyed by the first wheel with respective mouths arranged downwardly and towards respective nozzles.

Due to the fact that the path extends for an arch which is less than 360 degrees, only a certain number of jaws convey respective containers.

The manifold is adapted to feed the sterilizing agent only to a certain number of nozzles, i.e. to those nozzles which are below to the containers conveyed by the first wheel.

On the other hand, the manifold is configured for preventing the sterilizing fluid from flowing from the source to the nozzles, which are arranged below the empty containers, in order to avoid any waste of sterilizing agent.

The manifold of the known sterilizing unit substantially comprises:
- a stator which is stationary relative to the axis and which defines an axial first duct in fluid connection with the source;
- a rotor, which rotates about the axis together with the second wheel and defines a plurality of second ducts in fluid connection with respective nozzles; and
- a stationary mask, which allows the fluid connection between the first duct and a given number of second ducts and prevents the fluid connection between the first duct and the remaining second ducts.

In detail, the mask comprises an inlet stretch which is in permanent fluid connection with the first duct and an outlet stretch which is eccentric relative to the axis.

Due to the rotation of the rotor, only some second ducts - and therefore only some corresponding nozzles - are in fluidic connection with the outlet stretch of the mask. In particular, the mask puts in fluidic connection the source and only those nozzles which are arranged in the arch along which containers are conveyed by the first wheel

Furthermore, the known manifold comprises:
- a plurality of bearings, for example ball bearings, which rotatably support the rotor on the stator; and
- a plurality of sealing elements, which protect the bearings from the sterilizing agent.

In particular, the rolling bearings are radially interposed between a radially inner element of the rotor and a radially outer element of the stator.

The Applicant has found that, when the sealing elements are worn out, the sterilizing agent contacts and, therefore, corrodes the bearings.

In order to prevent the resulting galling of the rotor and the stator, the manifold is generally replaced quite often, for example each three or four months depending on the throughput of the sterilizing unit.

A need is felt within the sector to avoid the above-described galling and to accordingly increase the life-time of the manifold.

Furthermore, the mask is kept in position with the rotating part by an O-ring. Accordingly, when the mask is worn out, the sterilizing agent can flow also in the nozzles which are not arranged below the containers. This causes the waste of the sterilizing agent outside the arch of the path, along which the containers are conveyed.

A need is therefore felt within the industry to ensure that the wear of the rotor does not impair the sealing function of the mask.

It is an object of the present invention to provide a manifold for conveying a sterilizing fluid inside empty articles, which meets at least one of the above requirements.

The aforementioned object is achieved by the present invention as it relates to a manifold for conveying a sterilizing fluid inside empty articles, as claimed in claim 1.

One preferred embodiments is hereinafter disclosed for a better understanding of the present invention, by way of non-limitative example and with reference to the accompanying drawings, in which:
- Figure 1 is a frontal view, with some sectioned components, of a sterilizing unit comprising a manifold in accordance with the present invention;
- Figure 2 is a top view of the sterilizing unit of Figure 1;
- Figure 3 is a first transversal section of the manifold of Figure 1;
- Figure 4 is a second transversal section of the manifold of Figure 1; and
- Figure 5 is a section of the manifold of Figures 2 to 4, taken along line V-V of Figure 3.

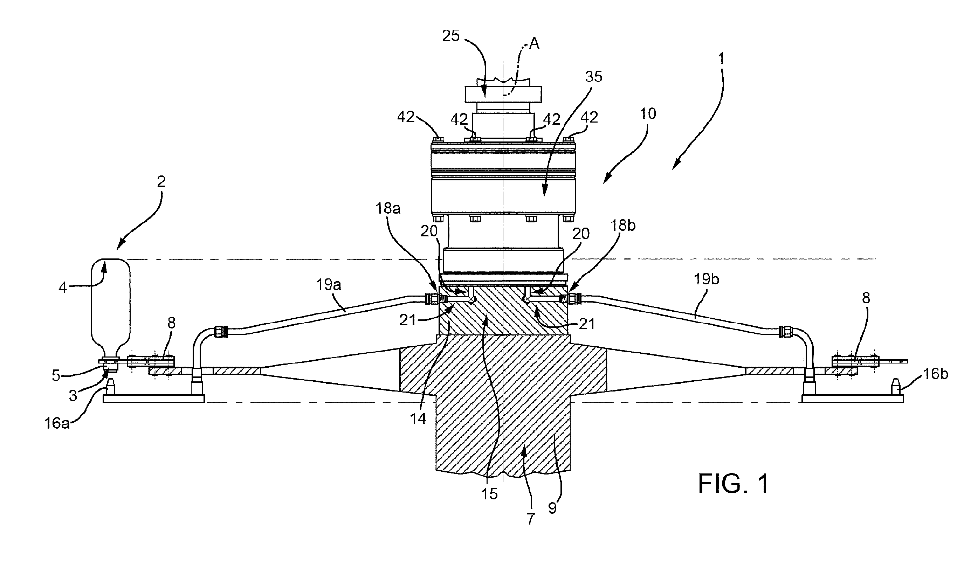

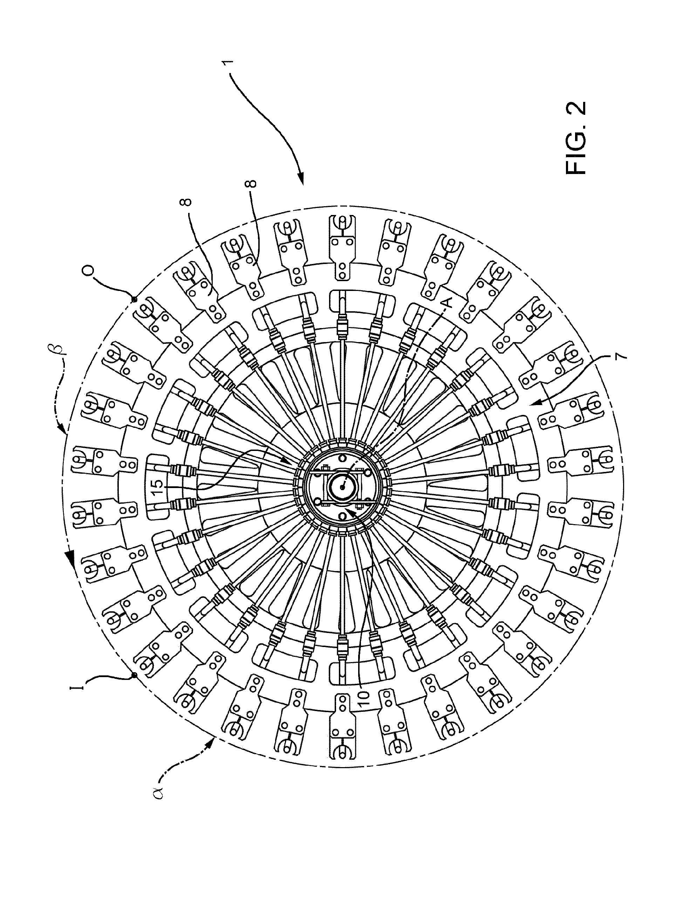

With reference to Figures 1 and 2, numeral 1 indicates a sterilizing unit for sterilizing empty containers 2 filled with a pourable food product.

Non-limitative examples of the pourable food product are fruit juices, tea, beer or energy drinks.

Unit 1 is adapted to inject a sterilizing agent, peracetic agent in the embodiment shown, inside empty containers 2 (shown only in Figure 1), before the latter are filled the food product.

Unit 1 is incorporated in an article-handling machine which may comprise one or more of the following:
- a rinsing unit (not-shown) for removing the sterilizing agent from empty containers 2 before the filling thereof and arranged downstream of unit 1;
- a capping unit (not-shown) for applying a plurality of caps onto respective filled containers 2; and
- a labelling unit (not-shown) for applying a plurality of labels onto respective empty or filled containers 2.

In the embodiment shown, each container 2 substantially comprises:
- a mouth 3 adapted to allow the filling/pouring of the same container 2;
- a bottom wall 4 opposite to mouth 3; and
- a neck 5 arranged close to mouth 3.

Containers 2 may be made of glass or plastic.

Unit 1 extends about an axis A, vertical in the embodiment shown, and substantially comprises:
- a tank filled with the sterilizing agent;
- a wheel 7 which rotates about axis A, and which comprises a plurality of pairs of jaws 8 for receiving, conveying and releasing respective containers 2;
- a distributor wheel 15 which rotates about axis A together with wheel 7, and comprises a plurality of peripheral nozzles 16a, 16b arranged in axial correspondence with and below respective jaws 8; and
- a manifold 10, which selectively creates a fluidic connection between the tank and nozzles 16a and prevents the fluidic connection between the tank and second nozzles 16b.

In detail, wheel 7 comprises:
- a hub 9; and
- a plurality of angularly equi-spaced arms, which radially protrude from hub 9 and to which the pairs of jaws 8 are hinged to respective spokes of wheel 7 about respective vertical axes parallel to axis A.

In detail, wheel 7 receives containers 2 at an inlet station I, releases containers 2 at an outlet station O, and conveys containers 2 along an arch-shaped path P (Figure 2).

Path P has centre on axis A and extends from station I and station O.

Path P extends about axis A for an arch α which is less than 360 degrees.

Wheel 7 also moves along an arch β, which extends from station O to station I.

The sum of arch α and arch β is 360 degrees.

Arch β is smaller than arch α.

Each container 2, when travelling along path P, is arranged with respective mouth 3, which is arranged above and faces respective nozzle 16a and which is arranged below respective wall 4.

Each pair of jaws 8 is movable between an open configuration in which they grip neck 5 of respective container 2 and a closed configuration in which they are detached from neck 5 of respective container 2.

In particular, jaws 8 are in the open configuration when they receive respective containers 2 at station I and when they release respective containers 2 at station O. Jaws 8 are in the closed configuration when they convey containers 2 along path P.

Jaws 8 travelling along path P grip respective containers 2 whereas jaws 8 travelling from station O from station I do not grip any container 2.

Distributor wheel 15 comprises, in turn, (Figure 1):
- a hub 14, which is axially interposed between manifold 10 and hub 9;
- a plurality of ducts 18a, 18b, which are defined by hub 14 and selectively connectable with the tank by means of manifold 10; and
- a plurality of conduits 19a, 19b which fluidly connect respective ducts 18a, 18b with respective nozzles 16a, 16b, and are arranged outside hub 14.

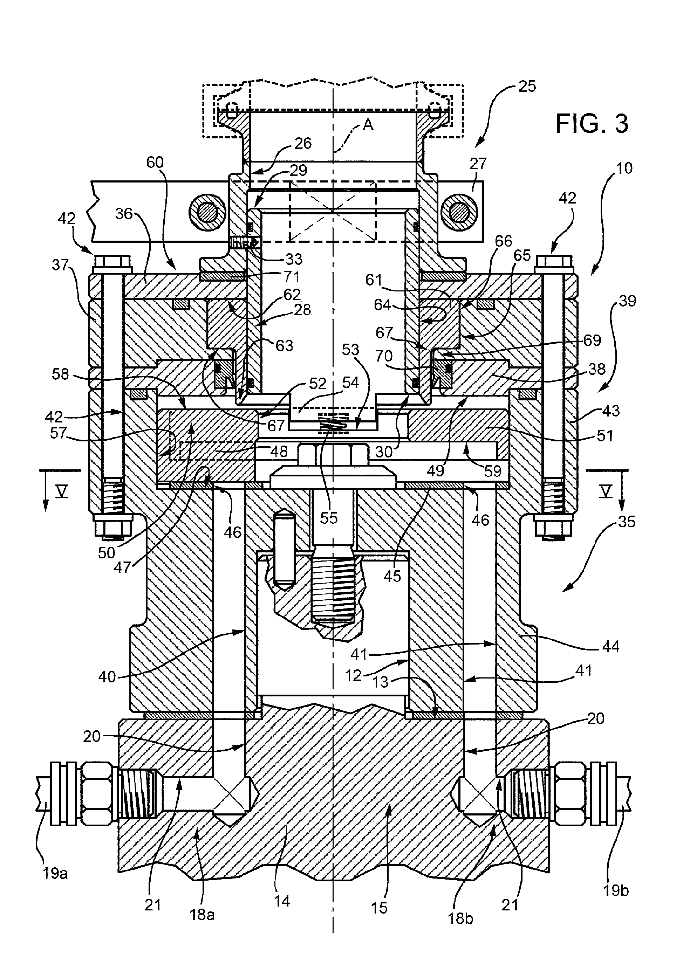

Hub 14 is bounded, on the side of manifold 10, by an annular surface 13 (Figure 3) from which an axial connecting element 12 protrudes along axis A.

Ducts 18a, 18b and conduits 19a, 19b are angularly equi-spaced relative to hub 14.

Conduits 19a, 19b protrude from hub 14 substantially along respective directions, which are transversal to axis A.

With reference to Figure 2, each duct 18a, 18b comprises an axial stretch 20 and a radial stretch 21, proceeding from manifold 10 towards conduits 19a, 19b.

Manifold 10 connects the tank with only nozzles 16a that are moving along arch α of path P and that, therefore, are arranged below respective containers 2.

Furthermore, manifold 10 prevents nozzles 16b - those which are travelling outside path P and from station O to station I along arch β - from being fluidly connected with the tank.

With reference to Figures 3 to 5, manifold 10 comprises:
- a stator 25 which is stationary relative to axis A and defines an axial tube 26 fluidly connected with the tank;
- a rotor 35 which rotates about axis A integrally with wheels 7, 15 and defines a plurality of ducts 40, 41 fluidly connected with respective nozzle 16a, 16b;
- a mask 50 for fluidly connecting tube 26 with ducts 40 - and therefore nozzle 16a - and for preventing the fluidic connection between tube 26 and ducts 41 - and therefore nozzle 16b -, on the basis of the angular position of rotor 35 and wheels 7, 15 about axis A; and
- supporting means 60 for supporting rotor 35 on stator 25.

In detail, stator 25 comprises, in turn,:
- tube 26;
- a blocking system 27 for blocking tube 26 to a fixed structure of unit 1;
- an axial tube 28 which is arranged inside tube 28, axially protrudes from tube 26 towards wheel 7, 15, and is connected to tube 26 by a radial grain 33 (visible in Figure 3).

Tube 28 is axially bounded by:
- an end 29 housed inside tube 26; and
- an end 30 opposite to end 29.

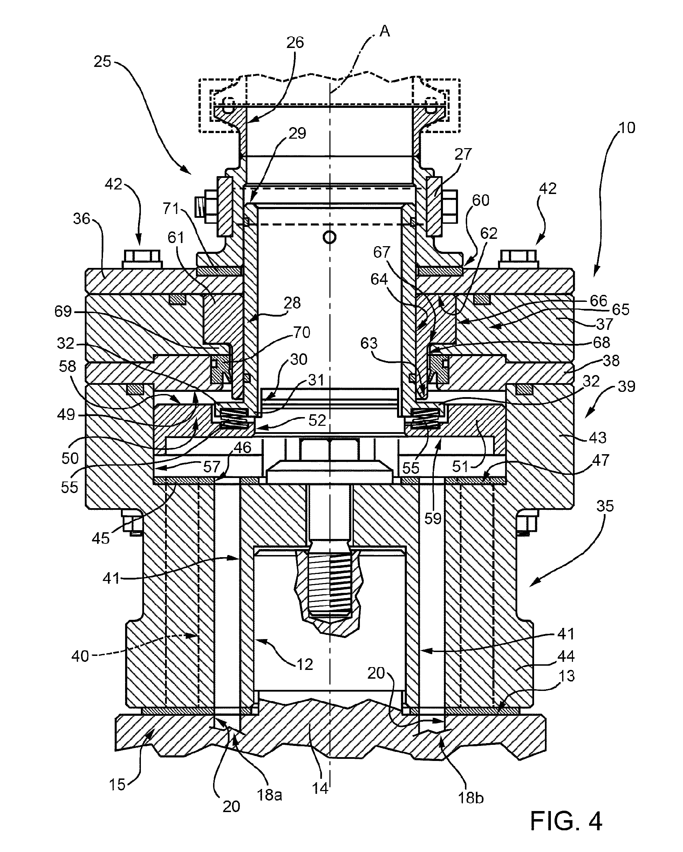

End 30 comprises, in particular, a ring 31 and a pair of appendices 32 radially protruding from ring 31 (Figure 3) on the opposite side of axis A. Appendices 32 are diametrically opposite to each other (Figure 4).

Ring 31 has the same area of end 29.

Still more precisely, end 29 and ring 31 have the same radial inner diameter and the area of tube 28 is constant, proceeding along axis A from end 29 to end 30.

Rotor 35 comprises, proceeding from the tank towards wheel 7, 15,:
- a flange 36 shaped as a circular crown having centre on axis A and an inner diameter which equals the outer diameter of tube 28;
- a flange 37;
- a flange 38 shaped as a circular crown;
- a cup-shaped body 39, which is connected to wheel 15 and defines ducts 40, 41.

Ducts 40, 41 are fluidly connected to respective ducts 18a, 18b and, therefore, to respective nozzle 16a, 16b.

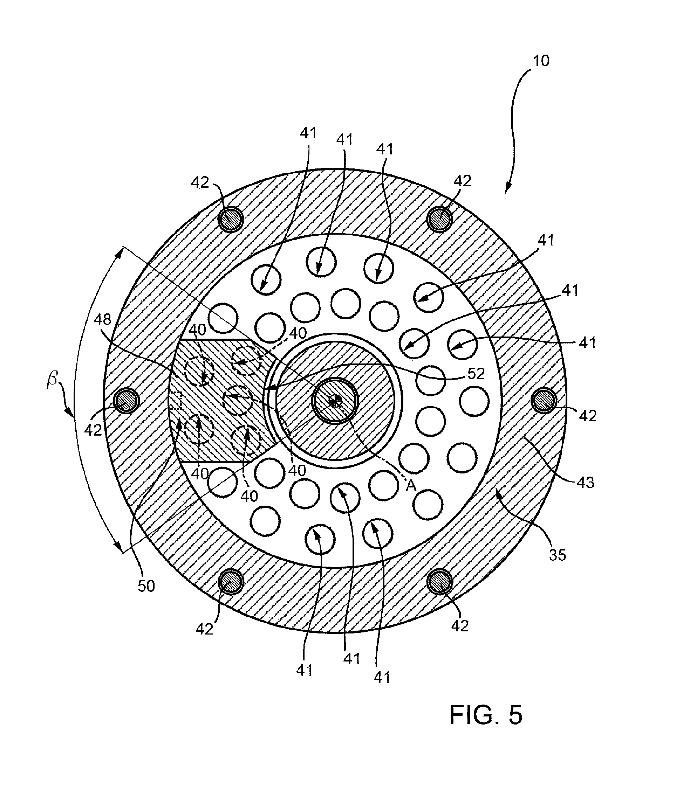

In the embodiment shown in Figure 5, ducts 40, 41 are arranged in two concentric series and ducts 40, 41 of each series are angularly equi-spaced about axis A. Furthermore, in the embodiment shown, ducts 40, 41 extend parallel to axis A.

Furthermore, rotor 35 comprises a plurality of angularly equi-spaced screws 42 which connect flanges 36, 37, 38 and body 39. Still more precisely, screws 42 are angularly equi-spaced about axis A and parallel to axis A.

Body 39 comprises, proceeding axially from flange 38 towards wheels 15 along axis A:
- a hollow portion 43;
- a main portion 44, which is screwed to connection element 12 of wheel 15 and defines ducts 40, 41; and
- a disk 45, which comprises a plurality of angularly equi-spaced holes 46 in angular correspondence with relative ducts 40, 41 and axially superimposed to ducts 40, 41.

More precisely, portion 44 is bounded, on the side of tubes 26, 28, by a surface 47 which extends radially to axis A and onto which disk 45 rests.

In the embodiment shown, disk 45 is made by a plastic material and may be replaced without replacing the whole body 39.

Manifold 10 also comprises a chamber 49 which is in fluid connection with tube 28 and may be selectively put in fluid connection with ducts 40, 41 by mask 50, on the basis of the angular position of rotor 35 about axis A.

Chamber 49 is radially bounded by portion 44 of rotor 35.

Furthermore, chamber 49 is axially bounded by end 30 of tube 28 and flange 38 on the side of tube 26, and by disk 45 on the side of wheels 7, 15.

Supporting means 60 comprises a support 61 which is radially interposed between flange 37 of rotor 35 and tube 28 of stator 25.

Advantageously, tube 28 is radially internal relative to flange 37.

In detail, support 61 is a plain bearing, a bushing in the embodiment shown.

Furthermore, support 61 is fitted to tube 28 and, therefore, is stationary about axis A.

Flange 37 rotates with friction onto support 61. Support 61 comprises:
- a top axial end surface 62 which axially cooperates with flange 36 of rotor 35;
- a bottom axial end surface 63 which is housed in chamber 49 and is subjected to the pressure of the sterilizing agent present in chamber 49; and
- an inner radial end surface 64 which extends between surfaces 62, 63 and which is fitted on tube 28.

The radial extension of surface 63 is smaller than the radial extension of surface 62.

Furthermore, support 61 comprises a radially outer surface 65, which is opposite to surface 64.

Surface 65 comprises:
- an axial stretch 66 adjacent to surface 62 and cooperating with flange 37;
- a shoulder 67 engaged with radial room by a radially inner protrusion 69 of flange 37; and
- an axial stretch 68 adjacent to surface 62.

In detail, flange 37 rotates with friction onto stretch 66 of support 61, and flange 36 rotates with friction onto surface 62 of support 61.

The axial size of support 61 is greater than radial size of support 61.

Supporting means 60 also comprise a further plain bearing, an annular friction element 71, in the embodiment shown. Friction element 71 is axially interposed between tube 26 and flange 36, and separated from tube 28 by a radial gap.

Flange 36 rotates with friction onto friction element 71 on the axial opposite side of support 61.

The radial size of friction element 71 is greater than axial size of same friction element 71.

Preferably, support 61 and/or friction element 71 are made in plastic material, in particular a plastic material with low sliding friction coefficient, high wear resistance, high chemical corrosion resistance and very low thermal expansion coefficient.

Support 61 and/or friction element 71 is/are made by plastic, preferably by Peek.

Manifold 10 also comprises a gasket 70, which is radially interposed between stretch 68 and a radially inner surface of flange 38. Gasket 70 comprises a lip which slides with friction on stretch 68 of surface 65, thus increasing the life-time of manifold 10.

Mask 50 is housed inside chamber 49 and extends annularly about axis A.

Furthermore, mask 50 is angularly connected in a fixed way to tube 28 about axis A and is elastically connected to tube 28 parallel to axis A. In particular, mask 50 is elastically pressed towards disk 45.

Mask 50 comprises:
- an annular disk 51, which defines an opening 52 coaxial with tube 28 and is fed by tube 28 with the sterilizing agent, and
- a shutter 48, which axially protrudes from disk 51 towards ducts 40, 41, is eccentric with respect to axis A and is pressed against disk 45.

Disk 51 comprises, on the side of tube 28, a pair of recesses 53 which are engaged by relative teeth 54 defined by end 30 of tube 28. In this way, mask 50 is angularly constrained to tube 28 and is, therefore, prevented from rotating about axis A.

Recesses 53 are opposite to each other about axis A.

Manifold 10 also comprises a pair of springs 55, helical in the embodiment shown, which are axially interposed between respective recess 53 and respective appendices 32 of end 30 of tube 28.

Springs 55 pre-load mask 50 towards disk 45.

Shutter 48 is arranged on one side only of axis A and extends about axis A for arch β, as shown in Figure 5.

Shutter 48 is pressed against disk 45 by the preloads of springs 55 and the pressure of the sterilizing agent inside chamber 49.

As rotor 35 and wheels 7, 15 rotate about axis A, ducts 40 (41) are temporary in the same angular position of and in axial correspondence with shutter 48 whereas ducts 41 (40) are temporary in the same angular position of and in axial correspondence with the remaining part of mask 50.

As a result, shutter 48 covers the holes of disk 45 which are in correspondence of ducts 40 (41). In this way, shutter 48 prevents the sterilizing agent from flowing from opening 52 to duct 40 (41) and, therefore, to nozzle 16a.

On the contrary, shutter 48 do not cooperate with ducts 41 (40), which are, at a given time, angularly staggered from it, thus leaving free the sterilizing agent of flowing from opening 52 to duct 41 (40).

Mask 50 is radially bound by a surface 57 which is fitted with a room inside portion 43 of rotor 35.

Mask 50 comprises:
- an axial end surface 58 which bounds disk 51 on the side of tubes 26, 28, and is axially spaced from flange 38; and
- a surface 59 opposite to surface 58, which bounds disk 51 on the side of disk 45 and is axially spaced from disk 45.

It is important to stress that surface 59 is not part of shutter 48.

Surface 58 extends for 360 degrees about axis A while surface 59 extends only for arch α about axis A, due to the presence of shutter 48.

As a result, surface 58 has an area A1 and surface 59 has an area A2, which is less than area A1 due to the presence of shutter 48.

Both surfaces 58, 59 are subjected to the pressure of the sterilizing agent flowing inside chamber 49.

Due to the fact that area A2 is smaller than area A1, the pressure of the sterilizing agent inside chamber 49 results in a downward force on mask 50, i.e. in a force directed towards disk 45.

In use, wheel 7 and distributor wheel 15 rotate integrally about axis A.

Wheel 7 is fed with containers 2 to be sterilized at station I, conveys containers 2 along arch α and outputs sterilized containers 2 at station O.

In particular, containers 2 are conveyed by wheel 7 along path P and arch α with respective mouths 3 arranged below respective bottom wall 4.

As distributor wheel 15 rotates, nozzles 16a are conveyed along arch α and are arranged below mouths 3 of respective containers 2. Differently, nozzles 16b are travelling from station O to station I along arch β, and are arranged below respective pairs of jaws 8 which are not gripping any container 2 (Figure 2).

Manifold 10 feeds the sterilizing agent to nozzles 16a only and prevents the sterilizing agent from reaching nozzles 16b.

In greater detail, stator 25 remains stationary relative to axis A while rotor 35 is driven in rotation by distributor wheel 15 about axis A.

As rotor 35 rotates, flange 37 rotates about axis A with friction on support 61.

Furthermore, flange 36 of rotor 35 rotates about axis A with friction on friction element 71 of stator 25.

The sterilizing agent flows from the tank to tubes 26, 28 and then reach chamber 49 and opening 52 of mask 50.

For each angular position of rotor 35 and distributor wheel 15 about axis A, mask 50 prevents the sterilizing agent from reaching those ducts 40 (41), which are temporary closed by shutter 48. In this way, mask 50 prevents the sterilizing agent from reaching nozzles 16b travelling along path β, which are associated to those ducts 40 (41) temporary closed by shutter 48.

On the other hand, for each angular position of rotor 35 and distributor wheel 15 about axis A, mask 50 allows the sterilizing agent to reach those ducts 41 (40), which are not covered by shutter 48 and that are, therefore, temporary in free communication with opening 52 and chamber 49. In this way, the sterilizing agent flows through ducts 41 (40) associated to nozzles 16a travelling along path α up to reach nozzles 16a themselves.

In greater detail, mask 50 is pressed against disk 45 and, therefore, ducts 40 (41) by springs 55 and by the downward force resulting from the pressure of sterilizing agent on surfaces 58, 59 of mask 50 having different areas A1, A2.

Furthermore, mask 50 is angularly constrained to tube 28 by the engagement of teeth 54 tube 28 and respective recesses 53 of disk 51.

Nozzles 16a can thus inject the sterilizing agent inside respective containers 2 travelling along path P and arranged along arch α.

In this way, containers 2 travelling along arch α are sterilized before that they are fed to the rinsing unit.

Nozzles 16b are prevented from injecting the sterilizing agent along arch β. In this way, there is no substantial waste of sterilizing agent.

From an analysis of the features of manifold 10 made according to the present invention, the advantages it allows to obtain are apparent.

In particular, flange 37 of rotor 35 is radially external relative to tube 28 of stator 25.

In other words, support 61 supports a radially external element - flange 37 - of rotor 35 on a radially internal element - tube 28 - of stator 25.

As a result, the pressure of the sterilizing agent applies on a reduced area of stator 25, namely the area of tube 28 - when compared with the known solution discussed in the introductory part of the present description where the stator is radially external with respect to the stator.

As a consequence, the design of manifold 10 results in a strong reduction of the loads acting on support 61.

This render possible to use a plain bearing subjected to sliding friction as support 61, instead of rolling bearings of the manifold discussed in the introductory part of the present description.

In other words, the radially internal position of tube 28 with respect to flange 37 results in the replacement of rolling bearings with components subject to sliding friction.

The reduction in the load acting on support 61 is further increased, due to the fact that end 29 and ring 31 of tube 28 have substantially the same area. Accordingly, the pressure of the sterilizing agent results in no substantial axial action on tube 28 and, therefore, on support 61.

In this way, manifold 10 no longer comprises supporting element, which can be corroded by the leaking of the sterilizing agent.

Accordingly, the risk of galling manifold 10 is lowered and the life-time of manifold 10 is highly increased when compared with the manifold described in the introductory part of the present description.

Furthermore, surface 62 of support 61 is larger than surface 63 of support 61.

Accordingly, in case of leakage of sterilizing agent through gasket 70, surface 62 is effective in containing the sterilizing agent inside manifold 10.

Moreover, the higher is the pressure of the sterilizing agent leaking from gasket 70, the higher is the upwards action which forces plain bearing 61 against flange 36.

In other words, any leakage of the sterilizing agent through gasket 70 presses plain bearing 61 against flange 36, thus increasing the sealing action deriving from the sliding contact between surface 62 of bearing 61 and flange 36.

Mask 50 is angularly fixed to tube 28 about axis A by the engagement of teeth 54 of tube 28 and respective recesses 53 of disk 51, and is axially connected to tube 28 by springs 55.

Accordingly, the position of mask 50 does not rely on any element, which undergoes wear as a result of the rotation of rotor 35 on stator 25.

In this way, the correct feeding of ducts 40 (41) and corresponding nozzles 16a only with the sterilizing agent is in no way affected by the friction and the wear due to the rotation of rotor 35 on stator 25.

Finally, area A1 of surface 58 is greater than area A2 of surface 59 of mask 50. Accordingly, the pressure of the sterilizing agent inside chamber 49 result in a downward force on mask 50, i.e. in a force directed against disk 45.

In this way, the greater is the pressure of the sterilizing agent, the greater is the downward force on shutter 48 of mask 50 which ensures that the sterilizing agent does no reach duct 40.

Finally, it is apparent that modifications and variants not departing from the scope of protection of the claims may be made to manifold 10.

## Claims

1. A manifold (10) for conveying a sterilizing fluid inside a plurality of empty articles (2), which are movable about an axis (A) and are arranged along an arch (α) having centre on said axis (A) and extending for less than 360 degrees about said axis (A), comprising:
- a stator (25), which is stationary relative to said axis (A) and which defines at least one duct (26, 28) for said sterilizing agent;
- a rotor (35), which may rotate relative to said stator (25) and defines at least one second duct (40, 41) and at least one third duct (41, 40) for said sterilizing fluid;
- supporting means (60, 61) radially interposed between a first element (28) of said stator (25) and a second element (37) of said rotor (35), and adapted to support said rotor (35) onto said stator (25); and
- a mask (50) fluidly interposed between said first duct (26, 28) and one (40) of said second duct (40, 41) and third duct (41, 40) on the basis of the angular position of said rotor (35) about said axis (A), so as to prevent said sterilizing agent from flowing inside said one (40) of said second duct (40, 41) and said third duct (41, 40); said mask (50) being configured to allow said sterilizing agent to flow from said first duct (26, 28) to the other one (41) of said second duct (40, 41) and said third duct (41, 40) on the basis of said angular position of said rotor (35) about said axis (A);
**characterized in that** said first element (28) is radially internal to said second element (37).

2. The manifold of claim 1, **characterized in that** said supporting means (60, 61) comprise a plain bearing (61) stationary relative to said axis (A) and onto which said second element (37) may rotate with friction about said axis (A).

3. The manifold of claim 2, **characterized in that** said plain bearing (61) comprises:
- a first surface (62), which axially cooperates with said rotor (25), and onto which said rotor (25) may rotate with friction about said axis (A); and
- a second surface (63), which is subject, in use, to the pressure of said sterilizing agent;
the radial extension of said first surface (62) being greater than the radial extension of said second surface (63).

4. The manifold of claim 2 or 3, **characterized in that** said plain bearing (61) is made in plastic material.

5. The manifold of any one of the foregoing claims, **characterized by** comprising a chamber (49) fluidly connected with said first duct (26, 28) and with said other one (41) of said second duct (40, 41) and third duct (41, 40).

6. The manifold of claim 5, **characterized in that** said mask (50) is arranged inside said chamber (49) and prevents the fluid connection between said chamber (49) and said other one (41) of said second duct (40, 41) and third duct (41, 40).

7. The manifold of claim 5 or 6, when depending on claim 3 or 4, **characterized in that** said second surface (63) is arranged inside said chamber (49).

8. The manifold of claim 7, **characterized in that** said bearing (61) comprises:
- a third surface (66), which extends about said axis (A) on the side of said first surface (62) and onto which said second element (37) may rotate with friction about said axis (A); and
- a fourth surface (67), which extends on the side of said second surface (63);
said manifold (10) further comprising a gasket (70) radially interposed between said rotor (35) and said fourth surface (67), and axially interposed between said first surface (61) and said second surface (62).

9. The manifold of claim 8, **characterized in that** said duct (26, 28) comprises a tube (28) connected to said plain bearing (61);
said tube (28) comprising a first axial end (29) through which said sterilizing agent may flow, and a second axial end (30, 31), opposite to said first axial end (29) and arranged inside said chamber (49);
the area of said first axial end surface (29) being substantially equal to the area of said second axial end (30, 31).

10. The manifold of any one of previous claim, **characterized by** comprising:
- connecting means (54, 53) for angularly connecting said mask (50) to said stator (25) and keeping said mask (50) angularly fixed about said axis (A); and
- elastic connecting means (55) for elastically connected said mask (50) and said stator (25) parallel to said axis (A).

11. The manifold of claim 10, when depending on claim 9, **characterized in that** said connecting means (54, 53) comprise:
- at least one recess (53) which is defined by one of said tube (28) and said mask (50); and
- at least one tooth (54) which is defined by the other one of said tube (28) and said mask (50), and is engaged by said tooth (54), so as to render said mask (50) and said stator (25) angularly integral to each other.

12. The manifold of claim 10 or 11, when depending on claim 9, **characterized by** comprising at least one spring (55) interposed between said tube (28) and said mask (50) and which elastically loads, in use, said mask (50) towards an element (44) of said rotor (25) defining said second duct (40, 41) and third duct (41, 40).

13. The manifold of anyone of claims 2 to 12, **characterized in that** said mask (50) comprises:
- a disk (51), which defines an opening (52) for said sterilizing agent and is fluid connection with said first duct (26, 28) and said other one (41) of said second duct (40, 41) and third duct (41, 40); and
- a shutter (48), which extends about axis (A) for a second arch (β) less than 360 degrees and prevents the fluid connection between said first duct (26, 28) and said one (40) of said second duct (40, 41) and third duct (41, 40).

14. The manifold of claim 13, when depending on any one of claims 5 to 12, **characterized in that** said mask (50) comprises:
- a first surface (58) which extends about said axis (A) for 360 degrees inside said chamber (49), bounds said disk (51) on the side of said first duct (26, 28), and is subjected, in use to the pressure of said sterilizing agent;
- a second surface (59) which extends about said axis (A) for said second arch (β) inside said chamber (49), bounds said disk (51) on the opposite axial side of said first duct (26, 28), and is subjected, in use to the pressure of said sterilizing agent;
said first surface (58) having a first area (A1) contactable by said sterilizing agent, which is greater than a second area (A2) of said second surface (59) contactable by said sterilizing agent, so that the pressure of said sterilizing agent thrusts, in use, said mask (50) towards said second duct (40) and third duct (41, 40).

15. A unit (1) for sterilizing a plurality of empty articles (2) before the filling thereof and which may move about an axis (A), comprising:
- a conveying wheel (6) which comprises a plurality of conveying elements (8) for conveying said articles (2) about said axis (A) and along said arch (α) from an inlet station (I) to an outlet station (O);
- an injecting wheel (15) which comprises a plurality of nozzles (16a, 16b) associated to respective conveying elements (8) and which are fluidly connected to said second duct (40, 41) and third duct (41, 40) respectively; and
- a manifold (10) according to any one of the foregoing claims and which is angularly integral with said injecting wheel (15) relative to said axis (A).
